Europäisches Patentamt

European Patent Office

Office européen des brevets

(19)

(11) Publication number: **0 117 934**
**B1**

(12) **EUROPEAN PATENT SPECIFICATION**

(45) Date of publication of patent specification: 20.07.88

(21) Application number: 83302888.9

(22) Date of filing: 20.05.83

(51) Int. Cl.⁴: **A 61 K 39/00,** A 61 K 39/385,
A 61 K 37/38, C 07 K 7/10

(54) Antigenic modification of polypeptides.

(30) Priority: 04.03.83 US 472190

(43) Date of publication of application:
12.09.84 Bulletin 84/37

(45) Publication of the grant of the patent:
20.07.88 Bulletin 88/29

(84) Designated Contracting States:
AT BE CH DE FR IT LI LU NL SE

(56) References cited:
GB-A-1 492 445
US-A-4 201 770
US-A-4 234 561
US-A-4 253 996
US-A-4 256 629
US-A-4 268 435
US-A-4 302 386
US-A-4 310 455
US-A-4 313 871
US-A-4 384 995

(73) Proprietor: The Ohio State University
200 Administration Building 190 North Oval Mall
Columbus Ohio 43210 (US)

(72) Inventor: Stevens, Vernon Cecil
5481 River Forest Road
Dublin Ohio 43017 (US)

(74) Representative: Cole, David John
Executive Liaison Services 54 Templemere
Weybridge Surrey KT13 9PB (GB)

(56) References cited:
CHEMICAL ABSTRACTS, vol. 83, no. 7, 18th
August 1975, page 331, no. 56613h, Columbus,
Ohio, USA M. DOERNER et al.: "Immunological
reactions of antibodies to human chorionic
gonadotropin (HCG) with HCG and its chemical
derivatives"

Note: Within nine months from the publication of the mention of the grant of the European patent, any person may
give notice to the European Patent Office of opposition to the European patent granted. Notice of opposition shall
be filed in a written reasoned statement. It shall not be deemed to have been filed until the opposition fee has been
paid. (Art. 99(1) European patent convention).

Courier Press, Leamington Spa, England.

## 0 117 934

**Description**

This invention relates to antigenic modification of polypeptides. More particularly, this invention relates to specific techniques for modification of polypeptides, to antigens for provoking the formation, by the immune system of an animal, of antibodies to exogenous proteins e.g. proteins found in viruses and other pathogenic organisms.

In our British Patent No. 1,567,764 and in our U.S. Patent No. 4,302,386 it is disclosed that hormones or other proteins found naturally in an animal can be chemically modified outside the body of the animal (a term which is used herein to include human beings) so that, when injected into the animal, the modified hormones or other proteins provoke the formation of antibodies which react not only with the chemically-modified hormone or other protein but also with the unmodified hormone or other protein found naturally in the animal, thereby reducing the level of the natural hormone or other protein in the body of the animal. Instead of using the entire hormone or other protein, the aforementioned patents also disclose the use of natural or synthetic fragments of such proteins in this so-called "isoimmunization" technique.

The aforementioned patents are mainly concerned with the application of their isoimmunization technique in controlling fertility, so that the protein which is modified is a reproductive hormone, such as Follicle Stimulating Hormone (FSH), lutenising Hormone (LH), Human Placental Lactogen (HPL), Human Prolactin and Human Chorionic Gonadotropin (HCG), or a peptide having an amino acid sequence corresponding to a part of one of these hormones. However, the aforementioned patents also disclose the applicability of the isoimmunization technique to other protein hormones, for example:

1. Gastrin, for the treatment of Zollinger-Ellison Syndrome;
2. Angiotension II, for the treatment of hypertension;
3. Growth hormone and somatomedian, for the treatment of diabetes and associated micro and macro vascular diseases;
4. Parathyroid hormone for the treatment of kidney stones;
5. Insulin and glucagon, for the treatment of hyperinsulinoma;
6. Thyroid stimulating hormone, for the treatment of hyperthyroidism;
7. Secretin, for the treatment of irritable bowel syndrome.

The aforementioned patents also disclose the use of modified polypeptides derived from chorionic gonadotropin, LH or FSH, or a fragment thereof, for use in treating certain carcinomas.

The main technique of chemical modification disclosed in the aforementioned patents is coupling of the hormone, other protein or fragment thereof to a large "carrier" molecule such as diphtheria toxoid, tetanus toxoid or a synthetic polymer; these carrier molecules are not endogenous to the animal to be treated. The aforementioned patents also describe polymerization of the hormone, other protein, or fragment thereof by reaction with a bifunctional organic reagent (for example a bi-functional imidoester such as dimethyl adipimidate, dimethyl suberimidate or diethyl malonimidate) or dimerization by oxidation of a thiol group to form a disulfide bridge. All these modes of chemical modification have disadvantages. Dimerization of the hormone, other protein or fragment via a disulfide bridge does not introduce exogenous material into the animal being treated but, since the chemically-modified antigen administered to the animal is only a dimer of a hormone, other protein or fragment which is not itself immunogenic to the animal, such dimers are rarely successful in provoking useful levels of antibodies. The modified polypeptides produced using carriers contain a very high proportion of non-endogenous material, and will usually provoke the formation of substantial quantities of antibodies to the carrier as well as to the hormone, other protein or fragment thereof. Although the formation of antibodies to the carrier may be sometimes be useful (for example, a vaccine based upon a HCG fragment coupled to diphtheria toxoid and intended for fertility control has the incidental advantage of also conferring protection against diphtheria), there are many occasions upon which it is not desirable to provoke the formation of relatively large quantities of antibodies to the carrier; for example, if one wishes to use a vaccine containing a modified polypeptide to treat a patient with a carcinoma or a serious viral infection, it may be desirable to avoid overstraining the patient's immune system by challenging it not only with the hormone, other protein or fragment thereof to which antibodies are desired, but also with the carrier.

In theory, perhaps the most promising of the modification techniques disclosed in the aforementioned patents is polymerization of the hormone, other protein or fragment thereof by reaction with a bifunctional reagent. This technique has, in theory, the advantage of introducing a relatively small proportion of non-endogenous material into the animal being treated (and even this relatively small proportion of non-endogenous material can be chosen so that it is not strongly immunogenic), while still providing a modified polypeptide large enough to be strongly immunogenic. Unfortunately, experiments have proved that straight-forward application of the bifunctional organic reagent polymerization technique to most hormones, other proteins or fragments thereof of practical interest produces very complicated mixtures of modified polypeptides having correspondingly complicated immunogenic properties. Furthermore, the immunogenic properties of the polymerized polypeptides thus produced are not readily reproduceable, but such reproduceability is essential in any material intended for pharmaceutical use, since the necessary tests of safety and efficiency cannot be performed on non-reproduceable material.

We have now found (though this knowledge is not in the published literature) that the reason for the

2

very complicated immunogenic properties and the lack of reproduceability present in polymers produced by the bifunctional organic reagent polymerization technique is that, notwithstanding the use of a bifunctional reagent, extensive cross-linking of the hormone, other protein fragment thereof occurs, such cross-linking presumably being due to the presence of free amino, thiol, carboxyl and perhaps other groups (the exact groups involved depending of course upon which groups the bifunctional organic reagent is designed to react with) at non-terminal positions on the hormone, protein or fragment thereof. Such cross-linking produces branching and three-dimensional structure in the resultant polymers. Not only does the relatively random cross-linking thus produced render the structure of the polymers themselves unpredictable and non-reproduceable, but such cross-linking may well alter the tertiary structure and shape of the hormone, other protein or fragment thereof being polymerized, thus affecting its immunogenic properties.

Accordingly, we have concluded that to produce useful modified polypeptides by the bifunctional organic reagent polymerization technique, it is essential to operate in such a way that coupling of the polypeptide fragments being polymerized occurs only at or near the terminals of the fragments, thus producing a true linear polymer substantially free of non-linear polymers of the fragments.

Accordingly, in one aspect this invention defines a polymeric polypeptide for provoking the formation, in the body of an animal, of antibodies to a protein, the polymeric polypeptide being characterized in that it comprises a linear polymer of polypeptide fragments, each of the fragments, in its monomeric form, being substantially non-immunogenic to the animal and having a molecular structure similar to a fragment of the protein to which antibodies are to be provoked, each adjacent pair of fragments in the linear polymer being connected via a residue of a bifunctional coupling agent, the linear polymer, after administration into the body of the animal, having a greater capacity to provoke the formation of the antibodies than the protein, the linear polymer being substantially free of non-linear polymers of the fragments.

In another aspect, this invention provides a method for producing a linear polymeric polypeptide for provoking the formation, in the body of an animal, of antibodies to one or more proteins which is substantially non-immunogenic to the animal, the method being characterized by:

(a) procuring a first peptide having a molecular structure similar to a fragment of the or one of the proteins;

(b) reacting this first peptide with a bifunctional coupling reagent while the first peptide is in a form having only a single site capable of reacting with the coupling reagent, this site being at or adjacent one of the termini of the first peptide, thereby causing one of the functional groups of the coupling reagent to react with the one site on the peptide;

(c) reacting the product of step (b) with a second peptide having a molecular structure similar to a fragment of the or one of the proteins while the second peptide is in a form having only a single site capable of reacting with the other functional group of the coupling reagent, this site being at or adjacent one of the termini of the second peptide, thereby forming a dimeric peptide wherein the first and second peptide are interconnected via a residue of the coupling reagent;

(d) reacting the resultant peptide with a bifunctional coupling reagent while the peptide is in a form having only a single site capable of reacting with the coupling reagent, this site being at or adjacent one of the termini of the peptide, thereby causing one of the functional groups of the coupling reagent to react with the one site on the peptide;

(e) reacting the product of step (d) with a further peptide having a molecular structure similar to a fragment of the or one of the proteins while this further peptide is in a form having only a single site capable of reacting with the other functional group of the coupling reagent, this site being at or adjacent one of the termini of the further peptide, thereby forming a polymeric peptide wherein the first, second and further peptides are interconnected via residues of the coupling reagent;

(f) repeating steps (d) and (e) until the desired polymer length has been achieved.

In another aspect, this invention provides a method for producing a modified polypeptide for provoking the formation, in the body of an animal, of antibodies to a protein which is substantially non-immunogenic to the animal, the method being characterized by:

a. procuring a first peptide having a molecular structure similar to a fragment of the protein, the first peptide not having an unblocked thiol group and having an unblocked amino group only at its N-terminal but no other unblocked amino group;

b. reacting the first peptide with an amino group activating agent, thereby producing an activated amino group at the N-terminal of the first peptide;

c. reacting the activated first peptide with a second peptide having a molecular structure similar to a fragment of the protein, the second peptide having a C-terminal cysteine bearing an unblocked thiol group but not having any other unblocked thiol groups, thereby coupling the N-terminal of the first peptide to the C-terminal of the second peptide;

d. reacting the resultant compound in a form having an unblocked amino group at its N-terminal but no other unblocked amino groups, and no unblocked thiol group, with an amino-group activating agent, thereby producing an activated amino-group at the N-terminal of the resultant compound;

e. reacting the activated compound produced in step (d) with a further peptide having a molecular structure similar to a fragment of the protein, this further peptide having a C-terminal cysteine bearing an unblocked thiol group, but not having any other unblocked thiol groups, thereby coupling the activated

3

N-terminal of the reactivated compound produced in step (d) to the C-terminal of the further peptide; and
f. repeating steps (d) and (e) until the desired polymer length has been achieved.

Finally, this invention provides a vaccine for provoking the formation, in the body of an animal, of antibodies to a protein, this vaccine being characterized in that it comprises a polymeric polypeptide of the invention and a vehicle comprising a mixture of mannide monooleate with squalane and/or squalene.

As already mentioned, the linear polymeric modified polypeptides of the invention comprise inert polymers of polypeptide fragments substantially free of non-linear polymers. In saying that the polypeptide fragments used in the linear polymeric polypeptide of the invention have a molecular structure similar to a fragment of the protein to which antibodies are to be provoked, we do not necessarily imply that the entire amino acid sequence of each fragment must correspond exactly to part of the protein to which antibodies are to be provoked; for example, in certain cases certain substitutions of amino acids may be possible without affecting the immunogenic character of the fragment. For example, our aforementioned U.S. Patent No. 4,302,386 describes a polypeptide, designated Structure (IX), which is notionally derived from the beta subunit of HCG but in which the cysteine residue at the 110 position is replaced by alpha-aminobutyric acid. In particular, when it is desired to provoke the formation of antibodies to an endogenous protein, the polypeptide fragments used to form the appropriate modified polypeptide of the invention must have a molecular structure similar to a fragment of the endogenous protein which antibodies are to be provoked, this does not exclude the possibility that such fragments might actually be derived from a protein in a different species, since many proteins are either identical between species or differ from one another so little in amino acid sequence that considerable cross-reactivity exists between antibodies to the corresponding proteins in the two species. For example, as mentioned below, zona pellucida enzymes from a pig will, when injected into humans, produce antibodies which display considerable activity against human zona antigens. Accordingly, for example, if one wishes to form a modified polypeptide for provoking the formation of antibodies, in humans, to zona pellucida antigens, appropriate polypeptide fragments may be prepared from zona pellucida antigens of pigs. Also, the fragments used in the linear polymeric polypeptides of the invention may incorporate sequences of amino acids having no counterpart in the sequence of the protein from which the fragment is notionally derived. Again, for example, our aforementioned U.S. Patent No. 4,302,386 describes certain polypeptide fragments, designated Structure (IV), (VIII), (IX), (X) and (XIV) which are notionally derived from the beta subunit of HCG but which incorporate spacer sequences comprising multiple proline residues.

It may at first appear surprising that a linear polymer of a polypeptide, the monomeric form of which is effectively non-immunogenic to an animal, can be immunogenic to the same animal. It is believed (though the invention is in no way limited by this belief) that the increase in immunogenicity upon polymerization is due to the increase in physical size of the molecule, which enables the molecule to be recognized much more easily by the animal's immune system. It can be shown that at least some monomeric polypeptides are very weakly immunogenic and cause the animal's immune system to produce detectable quantities of antibodies, which quantities, however are much too small to be effective. Immune systems are not well-adapted to recognize molecules as small as the small polypeptides when the polypeptides are present in polymeric form.

It will be apparent to those skilled in the art that the polypeptide fragments used in the linear polymeric polypeptides of the invention may be either natural (i.e. derived from natural proteins) or may be produced synthetically. Obviously, a synthetic polypeptide will perform in the same manner as a naturally occurring one in as much as the immune system of the animal to which the modified polypeptide is administered will react in exactly the same way to both. Also the fragments within each linear polymer may be the same or different and need not necessarily be derived from the same protein.

The linear polymeric polypeptides of the invention may be used to provoke the formation of antibodies to both endogenous and non-endogenous proteins by using appropriate polypeptide fragments to form the polymers. (The term "endogenous" is used herein to denote a protein which is native to the species to be treated, regardless of whether the antigen is endogenous to the particular individual animal being treated. Thus, for example, for present purposes a porcine sperm antigen is regarded as being endogenous to a sow even though obviously such a sperm antigen will not normally be present in the body of a sow). Further details of the use of non-endogenous proteins fragments of which may be utilized in the linear polymeric polypeptides of the invention is given below in connection with the discussion of the antigens of the invention. As regards endogenous proteins, fragments of which may be used to form the linear polymeric polypeptides of the invention, in general it may be stated that such endogenous proteins may include any of the proteins mentioned in our aforementioned U.S. Patent No. 4,302,386, as described above. Incidentally, it should be noted that the use of the linear polymeric polypeptides of the invention based upon growth hormone and/or somatomedian is not confined to diabetic patients. Thus, the linear polymeric polypeptides of the invention based upon these two hormones may be used to treat non-diabetic patients, such as persons suffering from acromegaly, who have excessive levels of growth hormone and/or somatomedian.

Particularly preferred linear polymeric polypeptides of the invention are those formed from fragments having a molecular structure similar to a fragment of Human Chorionic Gonadotropin, and in particular similar to a fragment of the beta-subunit thereof. Two particularly preferred fragments for use in the linear polymeric polypeptides of the invention are:

4

Asp - Asp - Pro - Arg - Phe - Gln - Asp - Ser - Ser - Ser - Ser - Lys - Ala - Pro - Pro - Pro - Ser - Leu - Pro - Ser - Pro - Ser - Arg - Leu - Pro - Gly - Pro - Ser - Asp - Thr - Pro - Ile - Leu - Pro - Gln - Cys (hereinafter designated fragment A);

and

Asp - His - Pro - Leu - Thr - Cys - Asp - Asp - Pro - Arg - Phe - Gln - Asp - Ser - Ser - Ser - Ser - Lys - Ala - Pro - Pro - Pro - Ser - Leu - Pro - Ser - Pro - Ser - Arg - Leu - Pro - Gyl - Pro - Ser - Asp - Thr - Pro - Ile - Leu - Pro - Gln - Cys.

Other HCG-derived fragments usable in the linear polymeric polypeptides of the invention include those of Structure (II)—(VIII), (VIIIa) and (IX)—(XIV) as set forth below, further details of the immunological nature of these fragments being given in the aforementioned U.S. Patent No. 4,302,386:

Structure (II)
Asp - Asp - Pro - Arg - Phe - Gln - Asp - Ser - Ser - Ser - Ser - Lys - Ala - Pro - Pro - Pro - Ser - Leu - Pro - Ser - Pro - Arg - Leu - Pro - Gly - Pro - Ser - Asp - Thr - Pro - Ile - Leu - Pro - Gln

Structure (III)
Gln - Asp - Ser - Ser - Ser - Ser - Lys - Ala - Pro - Pro - Pro - Ser - Leu - Pro - Ser - Pro - Ser - Arg - Leu - Pro - Gly - Pro - Ser - Asp - Thr - Pro - Ile - Leu - Pro - Gln

Structure (IV)
Cys - Pro - Pro - Pro - Pro - Pro - Pro - Ser - Asp - Thr - Pro - Ile - Leu - Pro - Gln

Structure (V)
Asp - Asp - Pro - Arg - Phe - Gln - Asp - Ser - Pro - Pro - Pro - Pro - Pro - Pro - Cys

Structure (VI)
Phe - Gln - Asp - Ser - Ser - Ser - Ser - Lys - Ala - Pro - Pro - Pro - Ser - Leu - Pro - Ser - Pro - Ser - Arg - Leu - Pro - Gly - Pro - Ser - Asp - Thr - Pro - Ile - Leu - Pro - Gln

Structure (VII)
Asp - Asp - Pro - Arg - Phe - Gln - Asp - Ser - Ser - Ser - Ser - Lys - Ala - Pro - Pro - Pro - Ser - Leu - Pro - Ser

Structure (VIII)
Asp - Asp - Pro - Arg - Phe - Gln - Asp - Ser - Pro - Pro - Pro - Cys - Pro - Pro - Pro - Ser - Asp - Thr - Pro - Ile - Leu - Pro - Gln

Structure (VIIIa)
Asp - Asp - Pro - Arg - Phe - Gln - Asp - Ser - Pro - Pro - Pro - Pro - Pro - Pro - Cys - Pro - Pro - Pro - Pro - Pro - Pro - Ser - Asp - Thr - Pro - Ile - Leu - Pro - Gln

Structure (IX)
Asp - His - Pro - Leu - Thr - Aba - Asp - Asp - Pro - Arg - Phe - Gln - Asp - Ser - Ser - Ser - Ser - Lys - Ala - Pro - Pro - Pro - Ser - Leu - Pro - Ser - Pro - Ser - Arg - Leu - Pro - Gly - Pro - Ser - Asp - Thr - Pro - Ile - Leu - Pro - Gln - Pro - Pro - Pro - Pro - Pro - Cys

Structure (X)
Asp - Asp - Pro - Arg - Phe - Gln - Asp - Ser - Ser - Ser - Ser - Lys - Ala - Pro - Pro - Pro - Ser - Leu - Pro - Ser - Pro - Ser - Arg - Leu - Pro - Gly - Pro - Ser - Asp - Thr - Pro - Ile - Leu - Pro - Gln - Pro - Pro - Pro - Pro - Pro - Pro - Cys

Structure (XI)
Asp - Asp - Pro - Arg - Phe - Gln - Asp - Ser - Ser - Ser - Ser - Lys - Ala - Pro - Pro - Pro - Ser - Leu - Pro - Ser - Pro - Ser - Arg - Leu - Pro - Gly - Pro - Ser - Asp - Thr - Pro - Ile - Leu - Pro - Gln - Cys

Structure (XII)
Thr - Cys - Asp - Asp - Pro - Arg - Phe - Gln - Asp - Ser - Ser - Ser - Ser - Lys - Ala - Pro - Pro - Pro - Ser - Leu - Pro - Ser - Pro - Ser - Arg - Leu - Pro - Gly - Pro - Ser - Asp - Thr - Pro - Ile - Leu - Pro - Gln

Structure (XIII)
Asp - His - Pro - Leu - Thr - Aba - Asp - Asp - Pro - Arg - Phe - Gln - Asp - Ser - Ser - Ser - Ser - Lys - Ala - Pro - Pro - Pro - Ser - Leu - Pro - Ser - Pro - Ser - Arg - Leu - Pro - Gly - Pro - Ser - Asp - Thr - Pro - Ile - Leu - Pro - Gln - Cys

Structure (XIV)

Cys - Pro - Pro - Pro - Pro - Pro - Pro - Asp - Asp - Pro - Arg - Phe - Gln - Asp - Ser - Ser - Ser -
Ser - Lys - Ala - Pro - Pro - Pro - Ser - Leu - Pro - Ser - Pro - Ser - Arg - Leu - Pro - Gly - Pro -
Ser - Asp - Thr - Pro - Ile - Leu - Pro - Gln

Obviously, if it is desired to use one of the above peptides which lacks a C-terminal cysteine as a second or later fragment in preparing the linear polymeric peptides of the invention, will be necessary to add a C-terminal cysteine to the peptide; appropriate methods for doing so are of course well known to those skilled in the field of polypeptide synthesis. Also, some of the above peptides will of course require blocking of non-terminal amino and/or thio groups before use.

The linear polymeric polypeptides of the invention based upon HCG-derived fragments are usable in exactly the same manner as the corresponding modified polypeptides in which the same fragments are coupled to carriers as described in our aforementioned U.S. Patent No. 4,320,386. Moreover, since this patent was written, further discoveries have been made concerning the association between HCG and immunologically similar materials and certain carcinomas. It appears (though the invention is in no way limited by this belief) that certain carcinomas exude chorionic gonadotropin or an immunologically-similar material on their surfaces, thereby presenting to the immune system of the host animal a surface, which, superficially, appears to be formed of material endogenous to the host animal, and which is thus relatively non-immunogenic. Because of this known association between certain carcinomas and chorionic gonadotropin or chorionic gonadotropin-like materials, the HCG-derived linear polymeric polypeptides of the invention are useful for the treatment of HCG and chorionic gonadotropin-association carcinomas. The same linear polymeric polypeptides of the invention are also of course useful for fertility control, as described in the aforementioned U.S. Patent.

The fragments used in the linear polymeric polypeptides of the invention may also include fragments of the zona pellucida and sperm antigens discussed in detail below.

As already mentioned, the linear polymeric peptides of the invention are produced by polymerizing fragments of the protein to which antibodies are to be provoked rather than the entire protein itself. This use of fragments rather than an entire proteins has important advantages (and similar advantages are secured by the use of fragments in the antigens and modified antigens of the invention discussed in detail below). It is well recognized by those skilled in immunology (see e.g. W. R. Jones, "Immunological Fertility Regulation", Blackwell Scientific Publications, Victoria, Australia (1982) pages 11 et seq.) that one of the greatest potential hazards of a vaccine, especially a contraceptive vaccine, is cross-reactivity with non-target antigens, producing what is essentially an artificially-induced autoimmune disease capable of causing immunopathological lesions in, and/or loss of function of, the tissues carrying the cross-reactive substances. Two possible mechanisms for such cross-reactivity are:

(a) Presence of shared antigenic determinants; a complex protein may contain components (amino-acid sequences) identical to those present in a non-target antigens;

(b) steric overlap between non-identical but structurally related parts of the protein and non-target antigens.

Obviously, the changes posed by both these modes of cross-reactivity may be lessened by using, in the linear polymeric polypeptides, antigens and modified antigens of the invention, a fragment of a complex protein rather than the whole protein. Since the fragment has a simpler sequence than the protein from which it is derived, there is less chance of shared antigenic determinants or steric overlap with non-target proteins. In particular, cross-reactions can often be avoided by using fragments derived from a portion of the target protein (i.e. the protein to which antibodies are to be provoked) which is not similar in sequence to the non-target but cross-reactive protein. In particular, as described in our aforementioned U.S. Patent No. 4,320,386, one of the major problems in provoking anitbodies to HCG is cross-reactivity of HCG antibodies with LH, this cross-reactivity being at least largely due to identity of amino acid sequence between LH and the 1—110 amino acid sequence of the beta subunit of HCG. Accordingly, when it is desired to form an HCG-derived linear polymeric polypeptide of the invention, the fragment used is preferably one having a molecular structure similar to part or all of the 111—145 sequence of the beta subunit of HCG, since it is only this 111—145 sequence of beta-HCG which differs from the corresponding sequence of LH. However, research indicates that the fragments used in the linear polymeric polypeptides of the invention may contain sequences corresponding to the 101—110 sequence of beta-HCG which is common to beta-HCG and LH without inducing the formation of antibodies reactive to LH. Thus, one can use in the linear polymeric polypeptides of the invention fragments containing part of or all of this common 101—110 sequence without causing cross-reactivity with LH. For example, Structure (II) above represents the 111—145 amino acid sequence of beta-HCG. If desired, a fragment having the 101—145 amino acid sequence of beta-HCG could be substituted for the fragment of Structure (II) in the linear polymeric polypeptides of the invention without substantially effecting the activity of the linear polymers and without causing cross-reactivity with LH.

Polymerization of the fragments to form the linear polymeric polypeptides of the invention may be effected in any manner for coupling peptide fragments to form linear polymers thereof known to those skilled in the art. The linear polymeric polypeptides of the invention may be divided into two distinct types. In the first type, the individual peptide fragments are linked head-to-tail by peptide linkages, so that the

6

whole polymer comprises solely the fragments themselves and does not contain any extraneous material. Although such pure polymers do have the advantage of not introducing any extraneous material into the body of the animal being treated, they are usually too expensive to be practical, since the necessary fragments (whether produced by total synthesis or cleavage of a natural protein) are themselves very expensive and substantial losses occur during the polymerization process. Furthermore, the head-to-tail coupling of the fragments, without any intervening residues, may produce immunological determinants which have no counterpart in the unpolymerized fragment. For example, if the fragment described above, comprising the 105—145 sequence of HCG, is polymerized by means of peptide linkages, a sequence:

Pro - Ile - Leu - Pro - Gln - Asp - His - Pro - Leu - Thr

will be produced at each junction between adjacent fragments, and this sequence may provoke the formation of antibodies which would not be produced by the fragment itself, and which may be undesirable. In colloquial terms, since there is no "punctuation" to tell the immune system of the recipient animal where one fragment begins and another ends, the animal's immune system may inadvertently start reading at the wrong residue and produce unwanted antibodies by running the sequences of adjacent fragments together. For this reason, in general, we do not recommend the use of linear polymers in which the fragments are connected by peptide polymers, though of course such linear polymers may be useful in certain instances.

Various methods of coupling polypeptide fragments via peptide bonds are known to those skilled in the art. For example, one fragment to be coupled may have its C-terminal carboxyl group blocked (e.g. by esterification) and be reacted with the other fragment, which has its N-terminal amino group blocked, at its carboxyl group activated by means of an activating agent. Obviously, blocking of non-terminal amino and carboxyl groups may be necessary. Also, as well known to those skilled in this field, it may be advantageous to attach one end of the polymer being produced to a support, such as polystyrene resin support, the polymer only being detached from the support after polymerization is completed.

In the second type of linear polymer polypeptide of the invention, the polypeptide fragments are connected to one another by means of residues derived from a bifunctional reagent used to effect polymerization of the fragments, so that the final linear polymer is an alternating linear polymer of polypeptide fragments and coupling reagent residues. Although this type of polymer necessarily introduces some extraneous material into the animal being treated, the proportion of extraneous material can be made considerably lower than it would be if the fragments were coupled to a large carrier, such as diphtheria toxoid. The coupling reagent, which is necessarily a bifunctional coupling reagent to produce a true linear polymer, can be chosen so that the residues it leaves in the polymer are not strongly immunogenic (so that they do not place the strain on the immune system of the recipient animal that, for example, a large carrier molecule such as diphtheria toxoid would) and the presence of these residues in the polymer has the advantage of substantially eliminating false immunological determinants produced by conjunction of the head of one fragment with the tail of an adjacent fragment, as discussed above.

To ensure that a true linear polymer is produced during the polymerization process, one terminal of a first polypeptide fragment is reacted with the bifunctional coupling reagent so that the coupling reagent reacts with a group present at or adjacent one terminal of the fragment; for example, the coupling reagent may react with a N-terminal amino group, a C-terminal carboxyl group or a free thiol group present on a C-terminal cysteine. Obviously, the nature of the coupling reagent used determines what group on the peptide reacts. In order to avoid any cross-linking and to ensure a reproduceable product, it is important that only one site on the first fragment be available for reaction with the coupling reagent so that the coupling reagent can only attach to the first fragment at this one site. As those skilled in this field are aware, if it is desired to use a fragment containing more than one group which could react with the coupling reagent, the excess sites may be blocked by attaching suitable protective groups thereto. The product formed by reaction of the first fragment with the coupling reagent is then reacted with a second fragment (which may be the same or different from the first fragment) having a single site available to react with the second reactive group of the bifunctional bicoupling reagent, thereby coupling the first and second fragments by a residue derived from the coupling reagent. Following any necessary purification of this dimeric product, it is then reacted with a further portion of a coupling agent which may be the same or different reagent from that used to effect the first coupling), thereby reacting the free terminal of either the first or second fragment with the coupling reagent. Naturally, it is important to ensure that only one site on the dimer is available for coupling to the coupling reagent, and as will be apparent to those skilled in the art, blocking or unblocking of potential reactive groups on the dimeric polypeptide may be necessary. The product of the reaction of the dimeric polypeptide with the coupling reagent is then reacted with a third fragment having only a single site available for reaction with the remaining reactive group of the coupling reagent, thereby producing a linear polymer containing three polypeptide fragments. Obviously, this process can be repeated until the desired size of linear polymer has been produced.

It will be apparent to those skilled in this field that the bifunctional coupling reagents used to prepare the linear polymeric polypeptides of the invention should be asymmetric i.e. they should have two functional groups which react with different groups on the fragments being polymerized, since, for example, if one attempted to react a bifunctional bicoupling reagent having two functional groups which

7

both reacted with amino groups with a first fragment having a single amino group, at least some of the first fragment would be dimerized via a residue derived from the bifunctional bicoupling reagent. Such dimerization may in theory be avoided by using a very large excess of the coupling reagent, but in practice it is undesirable to run the risk of producing even a small proportion of dimer. Similarly, in later stages of the polymerization process, it will be even more undesirable to use symmetric coupling reagents, thereby running the risk of dimerizing the partially formed polymers already produced. In the preferred process for producing the linear polymeric polypeptides in the invention already described, the polymer chain is begun with a first peptide having no unblocked thiol group and having an unblocked amino group only at its N-terminal (peptides containing thiol groups and/or amino groups other than at the N-terminal may of course be used if all these thiol and amino groups are blocked with any conventional blocking agent). This first peptide is then reacted with an amino group activating agent, a preferred activating agent for this purpose being 6-maleimido caproic acyl N-hydroxy succinimide ester (MCS); reaction of the peptide with this reagent is optimally effected at a pH of 6.6). The activating agent reacts with the amino group at the N-terminal of the first peptide to form an activated form of the first peptide; in the case of MCS, it is the ester portion of the reagent which reacts with the N-terminal group of the peptide. It is normally then necessary to remove excess activating agent before continuing the preparative process. Once the excess activating agent is removed, the activated first peptide is reacted with a second peptide having a C-terminal cysteine in a reduced state (i.e. having an unblocked 3-thiol group), thereby causing coupling of the N-terminal of the activated first peptide to the C-terminal of the second peptide via an activating agent residue. Desirably, the resultant dimer is purified as described in more detail below. Next, the dimer is again reacted with an amino-group activating agent and then with a second portion of the second peptide or with a third peptide, thereby producing a trimer. This procedure is repeated until the desired chain length has been achieved.

In order to secure reproduceable responses from the immune systems of treated animals, it is important that the linear polymeric polypeptides of the invention be used in the form pure polymers in which all the molecules contain the same number of fragments. To achieve such pure polymers, effective purification should be used after each polymerization step of the polymerization process. Because of the close chemical similarity between polymers containing different numbers of fragments, chemical purification is ineffective, so purification must be effected by physical methods. Gel filtration may be used if desired, but our preferred purification method is reverse-phase, high-pressure liquid chromatography, preferably using a molecular sieve as the solide phase.

In this method of forming linear polymers, the first and second peptides may be identical in chemical configuration except that in the first peptide the C-terminal cysteine has a blocked thiol group. The first two preferred fragments for forming linear polymeric polypeptides of the invention to form antibodies to HCG mentioned above may be described as (111—145)-Cys and (105—145)-Cys, where the figures refer to the amino acid sequence in the beta subunit of HCG. It will be appreciated that, when these fragments are to be used in forming linear polymeric polypeptides of the invention by the method just described, the lysine residue at position 122 must have its amino group blocked and, in the case of the (105—145)-Cys fragment, the non-terminal cysteine at position 110 must have its thiol group blocked, preferably with an acetamidomethyl group.

The linear polymeric peptides of the invention preferably contain from about 4 to about 14 fragments.

Finally, as mentioned above, this invention extends to a vaccine containing a modified polypeptide of the invention and a vehicle comprising a mixture of mannide monooleate with squalane and/or squalene. It has been found that this vehicle has the effect of increasing the quantity of antibodies provoked by the linear polymeric polypeptide of the invention when the vaccine is administered to an animal. To further increase the quantity of antibodies provoked by administration of the vaccine, it is advantageous to include in the vaccine an immunological adjuvant. The term "adjuvant" is used in its normal meaning to one skilled in the art of immunology, namely as meaning a substance which will elevate the total immune response of the animal to which the vaccine is administered i.e. the adjuvant is a non-specific immuno-stimulator. Preferred adjuvants are muramyl dipeptides, especially:

NAc-nor Mur-L · Ala-D · isoGln;
NAc-Mur-(6-0-stearoyl)-L · Ala-D · isoGln; or
NGlycol-Mur-L · αAbu-D · isoGln.

The vaccines of this invention may be administered parenterally to the animals to be protected; the usual modes of administration of the vaccine being intramuscular and sub-cutaneous injections. The quantity of vaccine to be employed will of course vary depending upon various factors, including the condition being treated and its severity. However, in general, unit doses of 0.1—50 mg in large mammals administered with one to five times the intervals of 1 to 5 weeks provide satisfactory results. Primary immunization may also be followed by "booster" immunization at 1 to 12 month intervals.

To prepare the vaccines of the invention, it is convenient to first mix the linear polymeric polypeptide of the invention with the muramyl dipeptide (or other adjuvant) and then to emulsify the resultant mixture in the mannide monooleate/squalene or squalane vehicle. Squalene is preferred to squalane for use in the vaccines of the invention, and preferably about 4 parts by volume of squalene and/or squalane are used per part by volume of mannide monooleate.

The animals to be treated with the linear polymeric polypeptides and vaccines of this invention include both humans and other animal species.

The following example is now given, though by way of illustration only, to show details of the preparation and use of a linear polymeric polypeptide of the invention.

Example

Fragment A described above (a fragment having an amino acid sequence corresponding to the 105—145 sequence of beta-HCG, with a cysteine residue added to the C-terminal thereof) was polymerized to form a hexamer. A first portion of fragment A had both its thiol groups (on the non-terminal cysteine at the position corresponding to position 110 of beta-HCG, and on its C-terminal cysteine) and its non-terminal amino group (on the lysine residue at the position corresponding to position 122 in beta-HCG) blocked. This blocked form of Fragment A was reacted with the bifunctional organic coupling reagent (or amino group activating agent) MCS in a buffered aqueous solution at pH 6.6, thereby reacting the ester portion of the MCS with the N-terminal amino group of the first portion of Fragment A. The resultant product was then reacted with a second portion of Fragment A, which was used in the same form as the first portion of Fragment A except that the C-terminal cysteine bore an unblocked thiol group, thereby reacting the remaining functional group of the MCS with the free thiol group on the second portion of Fragment A and producing a dimer in which the N-terminal of the first portion of Fragment A was coupled to the C-terminal of the second portion of Fragment A via an MCS residue. This dimer was then purified by gel filtration. The polymerization was then repeated in the same manner until a hexamer of Fragment A had been produced. Because the purification following each polymerization step was effected by gel filtration rather than by reverse-phase, high-pressure liquid chromatography, the hexamer was undoubtedly somewhat impure and contaminated by traces of pentamer, tetramer, etc., so that the results in the animal tests results described below could not be expected to be as good as would be produced using a pure hexamer.

To test the effectiveness of this hexameric polypeptide in provoking the formation of antibodies to HCG, the hexamer was formed into a vaccine using Complete Freunds' Adjuvant and injected into five rabbits. Each rabbit was given three injections of the vaccine intramuscularly at 3 week intervals, each injection containing 0.5 mg of the hexamer. Starting three weeks after the first injection, each rabbit was bled weekly and the level of antibodies to HCG in the blood determined. The following average values of antibody level were found (the figures in parenthesis represent the confidence limits i.e. average + or − standard error):

### TABLE

| Weeks after first injection | Antibody concentration (moles/liters$\times 10^{-10}$) |
|---|---|
| 3 | 5 (2—7) |
| 4 | 18 (13—22) |
| 5 | 30 (21—39) |
| 6 | 45 (30—60) |
| 7 | 58 (32—83) |
| 8 | 61 (34—86) |
| 9 | 77 (50—108) |
| 10 | 110 (53—>125) |
| 11 | 100 (50—>125) |
| 12 | 79 (30—119) |
| 13 | 53 (22—83) |

The, the above results show that even the crude hexamer preparation used in these experiments was much more strongly immunogenic than the very weakly immunogenic fragment from which it was derived.

**0 117 934**

**Claims**

1. A polymeric polypeptide for provoking the formation, in the body of an animal, of antibodies to a protein, the polymeric polypeptide being characterized in that it comprises a linear polymer of polypeptide fragments, each of the fragments, in its monomeric form, being substantially non-immunogenic to the animal and having a molecular structure similar to a fragment of the protein to which antibodies are to be provoked, each adjacent pair of fragments in the linear polymer being connected via a residue of a bifunctional coupling agent, the linear polymer, after administration into the body of the animal, having a greater capacity to provoke the formation of the antibodies than the protein, the linear polymer being substantially free of non-linear polymers of the fragments.

2. A polymeric polypeptide according to claim 1 wherein at least one of the fragments has an aminoacid sequence corresponding to part of the aminoacid sequence of the beta sub-unit of human chorionic gonadotropin.

3. A polymeric polypeptide according to claim 2 wherein at least one of the fragments has the aminoacid sequence below:

Asp - Asp - Pro - Arg - Phe - Gln - Asp - Ser - Ser - Ser - Ser - Lys - Ala - Pro - Pro - Pro - Ser - Leu - Pro - Ser - Pro - Ser - Arg - Leu - Pro - Gly - Pro - Ser - Asp - Thr - Pro - Ile - Leu - Pro - Gln - Cys (hereinafter designated fragment A);

and

Asp - His - Pro - Leu - Thr - Cys - Asp - Asp - Pro - Arg - Phe - Gln - Asp - Ser - Ser - Ser - Ser - Lys - Ala - Pro - Pro - Pro - Ser - Leu - Pro - Ser - Pro - Ser - Arg - Leu - Pro - Gyl - Pro - Ser - Asp - Thr - Pro - Ile - Leu - Pro - Gln - Cys

or one of the structures II to VIII, VIIIa and IX to XIV as defined herein

Structure (II)
Asp - Asp - Pro - Arg - Phe - Gln - Asp - Ser - Ser - Ser - Ser - Lys - Ala - Pro - Pro - Pro - Ser - Leu - Pro - Ser - Pro - Arg - Leu - Pro - Gly - Pro - Sěr - Asp - Thr - Pro - Ile - Leu - Pro - Gln

Structure (III)
Gln - Asp - Ser - Ser - Ser - Ser - Lys - Ala - Pro - Pro - Pro - Ser - Leu - Pro - Ser - Pro - Ser - Arg - Leu - Pro - Gly - Pro - Ser - Asp - Thr - Pro - Ile - Leu - Pro - Gln

Structure (IV)
Cys - Pro - Pro - Pro - Pro - Pro - Pro - Ser - Asp - Thr - Pro - Ile - Leu - Pro - Gln

Structure (V)
Asp - Asp - Pro - Arg - Phe - Gln - Asp - Ser - Pro - Pro - Pro - Pro - Pro - Pro - Cys

Structure (VI)
Phe - Gln - Asp - Ser - Ser - Ser - Ser - Lys - Ala - Pro - Pro - Pro - Ser - Leu - Pro - Ser - Pro - Ser - Arg - Leu - Pro - Gly - Pro - Ser - Asp - Thr - Pro - Ile - Leu - Pro - Gln

Structure (VII)
Asp - Asp - Pro - Arg - Phe - Gln - Asp - Ser - Ser - Ser - Ser - Lys - Ala - Pro - Pro - Pro - Ser - Leu - Pro - Ser

Structure (VIII)
Asp - Asp - Pro - Arg - Phe - Gln - Asp - Ser - Pro - Pro - Pro - Cys - Pro - Pro - Pro - Ser - Asp - Thr - Pro - Ile - Leu - Pro - Gln

Structure (VIIIa)
Asp - Asp - Pro - Arg - Phe - Gln - Asp - Ser - Pro - Pro - Pro - Pro - Pro - Pro - Cys - Pro - Pro - Pro - Pro - Pro - Pro - Ser - Asp - Thr - Pro - Ile - Leu - Pro - Gln

Structure (IX)
Asp - His - Pro - Leu - Thr - Aba - Asp - Asp - Pro - Arg - Phe - Gln - Asp - Ser - Ser - Ser - Ser - Lys - Ala - Pro - Pro - Pro - Ser - Leu - Pro - Ser - Pro - Ser - Arg - Leu - Pro - Gly - Pro - Ser - Asp - Thr - Pro - Ile - Leu - Pro - Gln - Pro - Pro - Pro - Pro - Pro - Cys

Structure (X)
Asp - Asp - Pro - Arg - Phe - Gln - Asp - Ser - Ser - Ser - Ser - Lys - Ala - Pro - Pro - Pro - Ser - Leu - Pro - Ser - Pro - Ser - Arg - Leu - Pro - Gly - Pro - Ser - Asp - Thr - Pro - Ile - Leu - Pro - Gln - Pro - Pro - Pro - Pro - Pro - Pro - Cys

10

Structure (XI)

Asp - Asp - Pro - Arg - Phe - Gln - Asp - Ser - Ser - Ser - Ser - Lys - Ala - Pro - Pro - Pro - Ser - Leu - Pro - Ser - Pro - Ser - Arg - Leu - Pro - Gly - Pro - Ser - Asp - Thr - Pro - Ile - Leu - Pro - Gln - Cys

Structure (XII)

Thr - Cys - Asp - Asp - Pro - Arg - Phe - Gln - Asp - Ser - Ser - Ser - Ser - Lys - Ala - Pro - Pro - Pro - Ser - Leu - Pro - Ser - Pro - Ser - Arg - Leu - Pro - Gly - Pro - Ser - Asp - Thr - Pro - Ile - Leu - Pro - Gln

Structure (XIII)

Asp - His - Pro - Leu - Thr - Aba - Asp - Asp - Pro - Arg - Phe - Gln - Asp - Ser - Ser - Ser - Ser - Lys - Ala - Pro - Pro - Pro - Ser - Leu - Pro - Ser - Pro - Ser - Arg - Leu - Pro - Gly - Pro - Ser - Asp - Thr - Pro - Ile - Leu - Pro - Gln - Cys

Structure (XIV)

Cys - Pro - Pro - Pro - Pro - Pro - Pro - Asp - Asp - Pro - Arg - Phe - Gln - Asp - Ser - Ser - Ser - Ser - Lys - Ala - Pro - Pro - Pro - Ser - Leu - Pro - Ser - Pro - Ser - Arg - Leu - Pro - Gly - Pro - Ser - Asp - Thr - Pro - Ile - Leu - Pro - Gln

4. A method for producing a linear polymeric polypeptide for provoking the formation, in the body of an animal, of antibodies to one or more proteins which are substantially non-immunogenic to the animal, the method being characterized by:

(a) procuring a first peptide having a molecular structure similar to a fragment of the or one of the proteins;

(b) reacting this first peptide with a bifunctional coupling reagent while the first peptide is in a form having only a single site capable of reacting with the coupling reagent, this site being at or adjacent one of the termini of the first peptide, thereby causing one of the functional groups of the coupling reagent to react with the one site on the peptide;

(c) reacting the product of step (b) with a second peptide having a molecular structure similar to a fragment of the or one of the proteins while the second peptide is in a form having only a single site capable of reacting with the other functional group of the coupling reagent, this site being at or adjacent one of the termini of the second peptide, thereby forming a dimeric peptide wherein the first and second peptides are interconnected via a residue of the coupling reagent;

(d) reacting the resultant peptide with a bifunctional coupling reagent while the peptide is in a form having only a single site capable of reacting with the coupling reagent, this site being at or adjacent one of the termini of the peptide, thereby causing one of the functional groups of the coupling reagent to react with the one site on the peptide;

(e) reacting the product of step (d) with a further peptide having a molecular structure similar to a fragment of the or one of the proteins while this further peptide is in a form having only a single site capable of reacting with the other functional group of the coupling reagent, this site being at or adjacent one of the termini of the further peptide, thereby forming a polymeric peptide wherein the first, second and further peptides are interconnected via residues of the coupling reagent;

(f) repeating steps (d) and (e) until the desired length has been achieved.

5. A vaccine for provoking the formation, in the body of an animal, of antibodies to a protein, the vaccine being characterized in that it comprises a polymeric polypeptide according to any of claims 1 to 3 or prepared according to claim 4 and a vehicle comprising a mixture of mannide monooleate with Squalane and/or Squalene.

**Patentansprüche**

1. Ein polymeres Polypeptid, das die Bildung von Antikörpern gegen Proteine im Körper eines Lebewesens verursacht, wobei das polymere Polypeptid dadurch gekennzeichnet ist, daß es ein lineares Polymere aus Polypeptidfragmenten umfaßt, wobei jedes der Fragmente, in seiner monomeren Form, wesentlich nichtimmunogen gegen das Lebewesen ist, und daß es eine Molekülstruktur hat, die ähnlich einem Fragment des Proteins ist, gegen das Antikörper gebildet werden, wobei jedes benachbarte Fragmentpaar in dem linearen Polymer durch einen Rest eines bifunktionellen Kupplungsagens verbunden wird, und das lineare Polymer, nach Einbringen in den Körper des Lebewesens, eine größere Kapazität zur Verursachung der Bildung der Antikörper als das Protein hat, wobei das lineare Polymer wirklich frei von nichtlinearen Polymeren der Fragmente sei.

2. Ein polymeres Polypeptid gemäß Anspruch 1, in dem wenigstens eins der Fragmente eine Aminosäuresequenz hat, die einem Teil der Aminosäuresequenz der β-Untereinheite des menschlichen Choriongonadotropin entspricht.

3. Ein polymeres Polypeptide gemäß Anspruch 2, in dem wenigstens eins der Fragmente die Aminosäuresequenz hat, wie nachfolgend festgelegt:

11

Asp - Asp - Pro - Arg - Phe - Gln - Asp - Ser - Ser - Ser - Ser - Lys - Ala - Pro - Pro - Pro - Ser - Leu - Pro - Ser - Pro - Ser - Arg - Leu - Pro - Gly - Pro - Ser - Asp - Thr - Pro - Ile - Leu - Pro - Gln - Cys (nachfolgend Fragment A genannt);

und

Asp - His - Pro - Leu - Thr - Cys - Asp - Asp - Pro - Arg - Phe - Gln - Asp - Ser - Ser - Ser - Ser - Lys - Ala - Pro - Pro - Ser - Leu - Pro - Ser - Pro - Ser - Arg - Leu - Pro - Gly - Pro - Ser - Asp - Thr - Pro - Ile - Leu - Pro - Gln - Cys;

oder eine der Strukturen II—VIII, VIIIa und IX—XIV, wie hier definiert.

Struktur (II)

Asp - Asp - Pro - Arg - Phe - Gln - Asp - Ser - Ser - Ser - Ser - Lys - Ala - Pro - Pro - Pro - Ser - Leu - Pro - Ser - Pro - Arg - Leu - Pro - Gly - Pro - Ser - Asp - Thr - Pro - Ile - Leu - Pro - Gln

Struktur (III)

Gln - Asp - Ser - Ser - Ser - Ser - Lys - Ala - Pro - Pro - Pro - Ser - Leu - Pro - Ser - Pro - Ser - Arg - Leu - Pro - Gly - Pro - Ser - Asp - Thr - Pro - Ile - Leu - Pro - Gln

Struktur (IV)

Cys - Pro - Pro - Pro - Pro - Pro - Pro - Ser - Asp - Thr - Pro - Ile - Leu - Pro - Gln

Struktur (V)

Asp - Asp - Pro - Arg - Phe - Gln - Asp - Ser - Pro - Pro - Pro - Pro - Pro - Pro - Cys

Struktur (VI)

Phe - Gln - Asp - Ser - Ser - Ser - Ser - Lys - Ala - Pro - Pro - Pro - Ser - Leu - Pro - Ser - Pro - Ser - Arg - Leu - Pro - Gly - Pro - Ser - Asp - Thr - Pro - Ile - Leu - Pro - Gln

Struktur (VII)

Asp - Asp - Pro - Arg - Phe - Gln - Asp - Ser - Ser - Ser - Ser - Lys - Ala - Pro - Pro - Pro - Ser - Leu - Pro - Ser

Struktur (VIII)

Asp - Asp - Pro - Arg - Phe - Gln - Asp - Ser - Pro - Pro - Pro - Cys - Pro - Pro - Pro - Ser - Asp - Thr - Pro - Ile - Leu - Pro - Gln

Struktur (VIIIa)

Asp - Asp - Pro - Arg - Phe - Gln - Asp - Ser - Pro - Pro - Pro - Pro - Pro - Pro - Cys - Pro - Pro - Pro - Pro - Pro - Pro - Ser - Asp - Thr - Pro - Ile - Leu - Pro - Gln

Struktur (IX)

Asp - His - Pro - Leu - Thr - Aba - Asp - Asp - Pro - Arg - Phe - Gln - Asp - Ser - Ser - Ser - Ser - Lys - Ala - Pro - Pro - Pro - Ser - Leu - Pro - Ser - Pro - Ser - Arg - Leu - Pro - Gly - Pro - Ser - Asp - Thr - Pro - Ile - Leu - Pro - Gln - Pro - Pro - Pro - Pro - Pro - Pro - Cys

Struktur (X)

Asp - Asp - Pro - Arg - Phe - Gln - Asp - Ser - Ser - Ser - Ser - Lys - Ala - Pro - Pro - Pro - Ser - Leu - Pro - Ser - Pro - Ser - Arg - Leu - Pro - Gly - Pro - Ser - Asp - Thr - Pro - Ile - Leu - Pro - Gln - Pro - Pro - Pro - Pro - Pro - Pro - Cys

Struktur (XI)

Asp - Asp - Pro - Arg - Phe - Gln - Asp - Ser - Ser - Ser - Ser - Lys - Ala - Pro - Pro - Pro - Ser - Leu - Pro - Ser - Pro - Ser - Arg - Leu - Pro - Gly - Pro - Ser - Asp - Thr - Pro - Ile - Leu - Pro - Gln - Cys

Struktur (XII)

Thr - Cys - Asp - Asp - Pro - Arg - Phe - Gln - Asp - Ser - Ser - Ser - Ser - Lys - Ala - Pro - Pro - Pro - Ser - Leu - Pro - Ser - Pro - Ser - Arg - Leu - Pro - Gly - Pro - Ser - Asp - Thr - Pro - Ile - Leu - Pro - Gln

Struktur (XIII)

Asp - His - Pro - Leu - Thr - Aba - Asp - Asp - Pro - Arg - Phe - Gln - Asp - Ser - Ser - Ser - Ser - Lys - Ala - Pro - Pro - Pro - Ser - Leu - Pro - Ser - Pro - Ser - Arg - Leu - Pro - Gly - Pro - Ser - Asp - Thr - Pro - Ile - Leu - Pro - Gln - Cys

Struktur (XIV)

Cys - Pro - Pro - Pro - Pro - Pro - Pro - Asp - Asp - Pro - Arg - Phe - Gln - Asp - Ser - Ser - Ser - Ser - Lys - Ala - Pro - Pro - Pro - Ser - Leu - Pro - Ser - Pro - Ser - Arg - Leu - Pro - Gly - Pro - Ser - Asp - Thr - Pro - Ile - Leu - Pro - Gln

4. Eine Methode zur Darstellung eines linearen polymeren Polypeptids, das, im Körper eines Lebewesens, die Bildung von Antikörpern gegen ein, oder mehrere, Proteine verursacht, die wirklich nicht immunogen gegen das Lebewesen sind, wobei die Methode gekennzeichnet sei durch:

a) Bereitstellen eines ersten Peptids, das eine Molekülstruktur hat, die ähnlich einem Fragment der, oder eines der, Proteine ist;

b) Reaktion dieses ersten Peptids mit einem bifunktionellen Kupplungsagens, wobei das erste Peptid in einer Form vorliegt, daß es nur eine einzige Seite hat, die in der Lage ist mit dem Kupplungsreagenz zu reagieren, und wobei diese Seite am, oder benachbart einem, terminalen Ende des ersten Peptids ist, so daß eine der funktionellen Gruppen des Kupplungsreagenz veranlaßt wird mit der einen Seite auf dem Peptid zu reagieren;

c) Reaktion des Produkts von Schritt b) mit einem zweiten Peptid, das eine Molekülstruktur hat, die ähnlich einem Fragment der, oder eines der, Proteine ist, wobei das zweite Peptid in einer Form vorliegt, daß es nur eine einzige Seite hat, die in der Lage ist mit der anderen funktionellen Gruppe des Kupplungsreagenz zu reagieren, und wobei diese Seite an einem, oder benachbart einem, der terminalen Enden des zweiten Peptids sei, so daß dadurch ein dimeres Peptid gebildet wird, in dem das erste und das zweite Peptid über einen Rest des Kupplungsreagenz verbunden sind;

d) Reaktion des resultierenden Peptids mit einem bifunktionellen Kupplungsreagenz, wobei das Peptid in einer Form vorliegt, daß es nur eine einzige Seite hat, die zur Reaktion mit dem Kupplungsreagenz in der Lage ist, und wobei diese Seite an einem, oder benachbart einem, der terminalen Enden des Peptids sei, so daß dadurch eine der funktionellen Gruppen des Kupplungsreagenz veranlaßt wird mit der einen Seite auf dem Peptid zu reagieren;

e) Reaktion des Produkts von Schritt d) mit einem weiteren Peptid, das eine Molekülstruktur hat, die ähnlich dem eines Fragments der, oder eines der, Proteine ist, wobei dieses weitere Peptid in einer Form vorliegt, daß es nur eine einzige Seite hat, die in der Lage ist mit der anderen funktionellen Gruppe des Kupplungsreagenz zu reagieren, und wobei diese Seite an einem, oder benachbart einem, der terminalen Enden des weiteren Peptids sei, so daß dadurch ein polymeres Peptid gebildet wird, in dem das erste, zweite und weitere Peptide über Reste des Kupplungsreagenz verbunden sind;

f) Wiederholung der Schritte d) und e) bis die gewünschte Länge erreicht worden ist.

5. Ein Vaccin zur Verursachung der Bildung von Antikörpern gegen ein Protein im Körper eines Lebewesens, wobei das Vaccin dadurch gekennzeichnet sei, daß es ein polymeres Polypeptid enthält, gemäß Anspruch 1—3, oder gemäß Anspruch 4 dargestellt wurde und einen Träger beinhaltet, welcher eine Mischung aus Mannidmonooleat mit Squalan und/oder Squalen enthält.

**Revendications**

1. Polypeptide polymère pour provoquer la formation, dans le corps d'un animal, d'anticorps à une protéine, le polypeptide polymère étant caractérisé en ce qu'il comprend un polymère linéaire de fragments de polypeptide, chacun des fragments, dans sa forme monomère, étant sensiblement non immunogénique vis-à-vis de l'animal et ayant une structure moléculaire semblable à un fragment de la protéine vis-à-vis de laquelle des anticorps doivent être créés, chaque paire adjacente de fragments dans le polymère linéaire étant reliée via un résidu d'un agent de couplage bifonctionnel, le polymère linéaire, après administration dans le corps de l'animal, ayant une capacité plus grande de provoquer la formation des anticorps que la protéine, le polymère linéaire étant sensiblement dépourvur de polymères non linéaires des fragments.

2. Polypeptide polymère selon la revendication 1, dans lequel au moins un des fragments présente une séquence d'acides aminés correspondant à la partie de la séquence d'acides aminés de la sous-unité beta de la gonadotrophine chorionique humaine.

3. Polypeptide polymère selon la revendication 2, dans lequel au moins un des fragments présente la séquence d'acides aminés définie ci-dessous:

Asp - Asp - Pro - Arg - Phe - Gln - Asp - Ser - Ser - Ser - Ser - Lys - Ala - Pro - Pro - Pro - Ser - Leu - Pro - Ser - Pro - Ser - Arg - Leu - Pro - Gly - Pro - Ser - Asp - Thr - Pro - Ile - Leu - Pro - Gln - Cys (hereinafter designated fragment A);

and

Asp - His - Pro - Leu - Thr - Cys - Asp - Asp - Pro - Arg - Phe - Gln - Asp - Ser - Ser - Ser - Ser - Lys - Ala - Pro - Pro - Pro - Ser - Leu - Pro - Ser - Pro - Ser - Arg - Leu - Pro - Gyl - Pro - Ser - Asp - Thr - Pro - Ile - Leu - Pro - Gln - Cys;

or one de structure II à VIII, VIIIa et IX à XIV comme défini ci-dessous:

13

0 117 934

Structure (II)
Asp - Asp - Pro - Arg - Phe - Gln - Asp - Ser - Ser - Ser - Ser - Lys - Ala - Pro - Pro - Pro - Ser -
Leu - Pro - Ser - Pro - Arg - Leu - Pro - Gly - Pro - Ser - Asp - Thr - Pro - Ile - Leu - Pro - Gln

Structure (III)
Gln - Asp - Ser - Ser - Ser - Ser - Lys - Ala - Pro - Pro - Pro - Ser - Leu - Pro - Ser - Pro - Ser -
Arg - Leu - Pro - Gly - Pro - Ser - Asp - Thr - Pro - Ile - Leu - Pro - Gln

Structure (IV)
Cys - Pro - Pro - Pro - Pro - Pro - Pro - Ser - Asp - Thr - Pro - Ile - Leu - Pro - Gln

Structure (V)
Asp - Asp - Pro - Arg - Phe - Gln - Asp - Ser - Pro - Pro - Pro - Pro - Pro - Pro - Cys

Structure (VI)
Phe - Gln - Asp - Ser - Ser - Ser - Ser - Lys - Ala - Pro - Pro - Pro - Ser - Leu - Pro - Ser - Pro -
Ser - Arg - Leu - Pro - Gly - Pro - Ser - Asp - Thr - Pro - Ile - Leu - Pro - Gln

Structure (VII)
Asp - Asp - Pro - Arg - Phe - Gln - Asp - Ser - Ser - Ser - Ser - Lys - Ala - Pro - Pro - Pro - Ser -
Leu - Pro - Ser

Structure (VIII)
Asp - Asp - Pro - Arg - Phe - Gln - Asp - Ser - Pro - Pro - Pro - Cys - Pro - Pro - Pro - Ser - Asp -
Thr - Pro - Ile - Leu - Pro - Gln

Structure (VIIIa)
Asp - Asp - Pro - Arg - Phe - Gln - Asp - Ser - Pro - Pro - Pro - Pro - Pro - Pro - Cys - Pro - Pro -
Pro - Pro - Pro - Pro - Ser - Asp - Thr - Pro - Ile - Leu - Pro - Gln

Structure (IX)
Asp - His - Pro - Leu - Thr - Aba - Asp - Asp - Pro - Arg - Phe - Gln - Asp - Ser - Ser - Ser - Ser -
Lys - Ala - Pro - Pro - Pro - Ser - Leu - Pro - Ser - Pro - Ser - Arg - Leu - Pro - Gly - Pro - Ser -
Asp - Thr - Pro - Ile - Leu - Pro - Gln - Pro - Pro - Pro - Pro - Pro - Pro - Cys

Structure (X)
Asp - Asp - Pro - Arg - Phe - Gln - Asp - Ser - Ser - Ser - Ser - Lys - Ala - Pro - Pro - Pro - Ser -
Leu - Pro - Ser - Pro - Ser - Arg - Leu - Pro - Gly - Pro - Ser - Asp - Thr - Pro - Ile - Leu - Pro -
Gln - Pro - Pro - Pro - Pro - Pro - Pro - Cys

Structure (XI)
Asp - Asp - Pro - Arg - Phe - Gln - Asp - Ser - Ser - Ser - Ser - Lys - Ala - Pro - Pro - Pro - Ser -
Leu - Pro - Ser - Pro - Ser - Arg - Leu - Pro - Gly - Pro - Ser - Asp - Thr - Pro - Ile - Leu - Pro -
Gln - Cys

Structure (XII)
Thr - Cys - Asp - Asp - Pro - Arg - Phe - Gln - Asp - Ser - Ser - Ser - Ser - Lys - Ala - Pro - Pro -
Pro - Ser - Leu - Pro - Ser - Pro - Ser - Arg - Leu - Pro - Gly - Pro - Ser - Asp - Thr - Pro - Ile -
Leu - Pro - Gln

Structure (XIII)
Asp - His - Pro - Leu - Thr - Aba - Asp - Asp - Pro - Arg - Phe - Gln - Asp - Ser - Ser - Ser - Ser -
Lys - Ala - Pro - Pro - Pro - Ser - Leu - Pro - Ser - Pro - Ser - Arg - Leu - Pro - Gly - Pro - Ser -
Asp - Thr - Pro - Ile - Leu - Pro - Gln - Cys

Structure (XIV)
Cys - Pro - Pro - Pro - Pro - Pro - Pro - Asp - Asp - Pro - Arg - Phe - Gln - Asp - Ser - Ser - Ser -
Ser - Lys - Ala - Pro - Pro - Pro - Ser - Leu - Pro - Ser - Pro - Ser - Arg - Leu - Pro - Gly - Pro -
Ser - Asp - Thr - Pro - Ile - Leu - Pro - Gln

4. Procédé pour produire un polypeptide polymère linéaire pour provoquer la formation, dans le corps d'un animal, d'anticorps à une ou plusieurs protéines qui sont sensiblement non immunogéniques vis-à-vis de l'animal, le procédé étant caractérisé par les étapes consistant à:
(a) fournir un premier peptide ayant une structure moléculaire semblable à un fragment de la ou d'une des protéines;

14

(b) faire réagir ce premier peptide avec un réactif de couplage bifonctionnel tandis que le premier peptide est sous une forme ayant seulement un site unique capable de réagir avec le réactif de couplage, ce site étant à une des terminaisons du premier peptide ou adjacent à celle-ci, en provoquant ainsi la réaction d'un des groupes fonctionnels du réactif de couplage avec ledit site sur le peptide;

(c) faire réagir le produit de l'étape (b) avec un deuxième peptide ayant une structure moléculaire semblable à un fragment de la ou de l'une des protéines tandis que le deuxième peptide est sous une forme ayant seulement un site unique capable de réagir avec l'autre groupe fonctionnel du réactif de couplage, ce site étant à l'une des terminaisons du deuxième peptide ou adjacent à celle-ci, en formant ainsi un peptide dimère dans lequel les premier et deuxième peptides sont reliés via un résidu du réactif de couplage;

- d) faire réagir le peptide résultant avec un réactif de couplage bifonctionnel tandis que le peptide est sous une forme ayant seulement un site unique capable de réagir avec le réactif de couplage, ce site étant à l'une des terminaisons du peptide ou adjacent à celle-ci, en provoquant ainsi la réaction d'un des groupes fonctionnels du réactif de couplage avec ledit site sur le peptide;

e) faire réagir le produit de l'étape d) avec un autre peptide ayant une structure moléculaire semblable à une fragment de la ou de l'une des protéines tandis que cet autre peptide est sous une forme ayant seulement un site unique capable de réagir avec l'autre groupe fonctionnel du réactif de couplage, ce site étant à une des terminaisons de l'autre peptide ou adjacent à celle-ci, en formant ainsi un peptide polymère dans lequel les premier, deuxième et autre peptides sont reliés via des résidus du réactif de couplage;

f) répéter les étapes d) et e) jusqu'à ce que la longueur souhaitée soit atteinte.

5. Vaccin pour provoquer la formation, dans le corps d'un animal, d'anticorps à une protéine, le vaccin étant caractérisé en ce qu'il comprend un polypeptide polymère selon l'une quelconque des revendications 1 à 3 ou est préparé selon la revendication 4, et un excipient comprenant un mélange de monooléate de mannide avec du squalane et/ou du squalène.